# EUROPEAN PATENT SPECIFICATION

(11) **EP 2 545 853 B1**
(45) Date of publication and mention of the grant of the patent: **25.10.2017**
(21) Application number: 11753263.0
(22) Date of filing: 03.03.2011
(51) Int. Cl.: A61B 5/1486, G01N 27/28, G01N 27/327, G01N 27/416

(54) **ELECTROCHEMICAL SENSOR**
ELEKTROCHEMISCHER SENSOR
CAPTEUR ÉLECTROCHIMIQUE

(30) Priority: 09.03.2010 JP 2010052411
(43) Date of publication of application: 16.01.2013
(73) Proprietor: ARKRAY, Inc., Minami-ku Kyoto-shi Kyoto 601-8045 (JP)
(72) Inventor: TSUKADA, Masashi, Kyoto-shi, Kyoto 602-0008 (JP)
(74) Representative: Piésold, Alexander James
(86) International application number: PCT/JP2011/054936
(87) International publication number: WO 2011/111604

(56) References cited:
- WO-A1-2008/105373
- WO-A2-2009/042631
- JP-A- 5 503 580
- JP-A- 2001 174 432
- JP-A- 2006 346 454
- JP-A- 2009 136 526
- US-A1- 2009 321 257
- US-B1- 6 284 478
- US-B2- 6 561 989

## Description

### TECHNICAL FIELD

The present invention relates to a sensor which measures a specified composition contained in an analyte.

### BACKGROUND ART

In a subcutaneous indwelling type glucose sensor, a plurality of electrodes is disposed in a minute area, a reagent containing an enzyme is deployed over the electrodes, and an external portion of the glucose sensor is covered with a plurality of polymers (e.g., Patent document 1).

In the subcutaneous indwelling type glucose sensor, a measurement target analyte is an interstitial fluid existing outside cells of subcutaneous tissues but does not exist amply unlike a blood. Glucose molecules existing in the analyte reach a detection layer where the enzyme exists via a sensor external layer film. A concentration of the glucose in a body fluid is estimated from current signals acquired from direct oxidization of an electron transfer mediator which transfers electrons generated by oxidizing the glucose reaching the detection layer with an enzyme and from the direct oxidization of hydrogen peroxide (H₂O₂) generated by oxidation reaction with electrodes.

At this time, if the analyte (the body fluid such as a blood and an interstitial fluid) does not sufficiently exist in the periphery of the glucose sensor, there is a possibility of causing such inconvenience that conduction between the electrodes cannot be attained, the acquired current signals do not get stabilized, and the glucose sensor does not respond due to being dried.

There is a method (e.g., Patent document 2) of acquiring the signals that are as high as possible from a small quantity of analyte by enlarging an area of the electrode in a way that forms a sensor base material in a geometrical shape. Another method is a method (e.g., Patent document 3) of introducing an analyte liquid into an interior of an insulating cylinder by configuring a cylindrical sensor in a manner that covers a wire type sensor with an electric insulator. Still another method is a method (e.g., Patent document 4) of forming a conductive material in a groove provided in a substrate. US2009/0321257 A1 discloses a biosensor that detects a target substance contained in a liquid sample, wherein the biosensor comprises a substrate, an electrode provided on the substrate, a reagent enzyme formed on a surface of the electrode, an external layer film provided on the substrate so as to cover the electrode and the reagent enzyme and a groove formed in the substrate.

### PRIOR ART DOCUMENTS

### PATENT DOCUMENTS

PATENT DOCUMENT 1: U.S. Patent No. 6119028
PATENT DOCUMENT 2: Japanese Unexamined Patent Application Publication No.2009-532700
PATENT DOCUMENT 3: U.S. Patent No. 6284478
PATENT DOCUMENT 4: U.S. Patent No. 6134461
PATENT DOCUMENT 5: Japanese Unexamined Patent Application Publication No.2010-532269

### SUMMARY OF THE INVENTION

### PROBLEMS TO BE SOLVED BY THE INVENTION

In the subcutaneous tissues, the interstitial fluid does not plentifully exist unlike the blood. On the occasion of measuring a concentration of a specified composition (e.g., glucose) contained in the analyte such as the interstitial fluid that does not amply exist within the subcutaneous tissues, there is a case of being hard to ensure a quantity of analyte needed for the measurement. In the case of measuring the specified composition contained in the analyte that does not plentifully exist within the subcutaneous tissues, a sufficient measurement result is not acquired as the case may be due to an electrode surf ace not being wetted with the analyte. Further, in the case of measuring the specified composition at a part where only a small quantity of analyte exists, the sufficient measurement result is not obtained in some cases due to the electrode surface not being wetted with the analyte. The present invention aims at restraining depletion of the analyte on the electrode surface by increasing a quantity of supply of the analyte to the electrode surface.

### MEANS FOR SOLVING THE PROBLEMS

The present invention adopts the following means in order to solve the problems given above. There is provided an electrochemical sensor comprising: a substrate; an electrode provided on the substrate; a reagent enzyme formed on a surface of the electrode; an external layer film provided on the substrate so as to cover the electrode and the reagent enzyme; and a groove formed in at least a part of the substrate in a direction of the electrode of the substrate, the groove having an interior for the introduction of analyte and for advancement of the analyte to contact the external layer film covering the electrode, so as to permeate the interior of the external layer film and be supplied onto the surface of the electrode. According to present invention, the groove is formed at least a part of the substrate in the direction of providing the electrode on the substrate, and hence, when the analyte exists in the periphery of the electrochemical sensor, it follows that the analyte is introduced into an interior of the groove of the substrate. The analyte introduced into the interior of the groove of the substrate advances through the interior of the groove of the substrate in the direction of providing the electrode on the substrate. The analyte contacts the external layer film covering the electrode, then permeates the interior of the external layer film and is thus supplied onto the surface of the electrode on the substrate.

According to the present invention, the analyte is introduced into the interior of the groove of the substrate, permeates the interior of the external layer film and can be supplied onto the surface of the electrode on the substrate via the external layer film. Accordingly, it is feasible to increase the quantity of supply of the analyte onto the surface of the electrode provided on the substrate. As a result, the analyte on the surface of the electrode provided on the substrate can be restrained from being depleted.

In the electrochemical sensor of the present invention, the electrode may be provided on the substrate so that at least a part of the electrode is positioned upwardly of the groove. The electrode is provided on the substrate so that at least the part of the electrode is positioned upwardly of the groove, whereby the analyte comes into contact with the electrode and is supplied onto the surface of the electrode on the substrate. According to the present invention, the analyte is introduced into the interior of the groove of the substrate and is made to contact the electrode, thereby enabling the analyte to be supplied onto the surface of the electrode on the substrate. In the electrochemical sensor of the present invention, the electrode may be provided, on the substrate, adjacent to the groove. The electrode is provided, on the substrate, adjacent to the groove, whereby the analyte is supplied onto the surface of the electrode on the substrate from the direction of the side surface of the electrode. According to the present invention, the analyte is introduced into the interior of the groove of the substrate and can be thereby supplied onto the surface of the electrode on the substrate from the direction of the side surface of the electrode.

In the electrochemical sensor of the present invention, the substrate may be formed with a plurality of grooves. According to the present invention, the substrate is formed with the plurality of grooves, whereby the analyte is introduced into the interiors of the plurality of grooves of the substrate, permeates the interior of the external layer film and can be supplied onto the surface of the electrode on the substrate via the external layer film. Moreover, according to the present invention, the substrate is formed with the plurality of grooves, whereby the analyte is introduced into the interiors of the plurality of grooves of the substrate, contacts the electrode and can be supplied onto the surface of the electrode on the substrate.

In the electrochemical sensor of the present invention, a plurality of electrodes may be provided on the substrate. The plurality of electrodes is provided on the substrate, thereby enabling the electrochemical sensor to continue the measurement even if a malfunction occurs in one electrode. In the electrochemical sensor of the present invention, the groove of the substrate may be formed in a concave shape. The concave shape includes a quadrangular shape, a semicircular shape, a semielliptical shape, a pyramid shape, etc. In the electrochemical sensor of the present invention, the groove may be formed in a way that extends from an end portion of the substrate toward the direction of the electrode of the substrate. In the electrochemical sensor of the present invention, at least two lines of grooves in the plurality of grooves may be connected to each other. The electrochemical sensor of the present invention may be used in the way of being subcutaneously indwelled.

Further, an electrochemical sensor of the present invention includes a substrate, an electrode and a groove to be formed in at least a part of the substrate, wherein the electrode and an external layer film are provided in an interior of the groove. According to the present invention, the electrochemical sensor includes the groove formed in at least the part of the substrate, and therefore, when the analyte exists in the periphery of the electrochemical sensor, the analyte is introduced into the interior of the groove of the substrate. The analyte introduced into the interior of the groove of the substrate contacts the external layer film provided in the interior of the groove of the substrate, permeates the interior of the external layer film and is thus supplied onto the surface of the electrode provided in the interior of the groove of the substrate.

According to the present invention, the analyte is introduced into the interior of the groove of the substrate, permeates the interior of the external layer film and can be supplied onto the surface of the electrode provided in the interior of the groove of the substrate via the external layer film. It is therefore feasible to increase the quantity of supply of the analyte onto the surface of the electrode provided in the interior of the groove of the substrate. As a result, the analyte on the surface of the electrode provided in the interior of the groove of the substrate can be restrained from being depleted.

In the electrochemical sensor of the present invention, the external layer film may be provided in the interior of the groove so as to cover the electrode. In the electrochemical sensor of the present invention, the groove of the substrate may be formed in the concave shape. The concave shape includes the quadrangular shape, the semicircular shape, the semielliptical shape, the pyramid shape, etc. In the electrochemical sensor of the present invention, the groove may be formed in a way that extends from one end portion of the substrate up to the other end portion of the substrate. In the electrochemical sensor of the present invention, the groove may be formed in a longitudinal direction of the substrate. The electrochemical sensor of the present invention may be used in the way of being subcutaneously indwelled.

### EFFECTS OF THE INVENTION

According to the present invention, the analyte on the electrode surface can be restrained from being depleted by increasing the quantity of supply of the analyte to the electrode surface.

### BRIEF DESCRIPTION OF THE DRAWINGS

[FIG. 1] FIG. 1 is a schematic view of a configuration of a composition continuous measuring apparatus 1 according to a first working example.
[FIG. 2] FIG. 2 is a perspective view of a whole electrochemical sensor 4 according to the first working example.
[FIG. 3] FIG. 3 is a sectional view of the electrochemical sensor 4 according to the first working example.
[FIG. 4] FIG. 4 is a sectional view of the electrochemical sensor 4 in the case of providing an electrode 22 upwardly of grooves 26 of a substrate 21.
[FIG. 5] FIG. 5 is a perspective view of the whole electrochemical sensor 4 in the case of providing a plurality of working electrodes 22A and a plurality of counter electrodes 22B on the substrate 21.
[FIG. 6] FIG. 6 is a sectional view of the electrochemical sensor 4 in the case of providing the plurality of working electrodes 22A and the plurality of counter electrodes 22B on the substrate 21.
[FIG. 7] FIG. 7 is a perspective view of the whole electrochemical sensor 4 according to a first modified example of the first working example.
[FIG. 8] FIG. 8 is a perspective view of the whole electrochemical sensor 4 according to a second modified example of the first working example.
[FIG. 9] FIG. 9 is a perspective view of the whole electrochemical sensor 4 according to a third modified example of the first working example.
[FIG. 10] FIG. 10 is a perspective view of a whole electrochemical sensor 30 according to a second working example.
[FIG. 11] FIG. 11 is a sectional view of the electrochemical sensor 30 according to the second working example.
[FIG. 12] FIG. 12 is a perspective view of the whole electrochemical sensor 30 in the case of providing a plurality of working electrodes 32A and a plurality of counter electrodes 32B in the interior of the groove 36 of the substrate 31.
[FIG. 13] FIG. 13 is a sectional view in the case of providing the plurality of working electrodes 32A and the plurality of counter electrodes 32B in the interior of the groove 36 of the substrate 31.
[FIG. 14] FIG. 14 is a perspective view of the whole electrochemical sensor 30 according to the first modified example of the second working example.
[FIG. 15] FIG. 15 is a perspective view of the whole electrochemical sensor 30 according to a second modified example of the second working example.

### MODE FOR CARRYING OUT THE INVENTION

An electrochemical sensor according to the present embodiment will hereinafter be described with reference to the drawings. Configurations in the following working examples are exemplifications, and the electrochemical sensor according to the embodiment is not limited to the configurations in the working examples.

### First Working Example

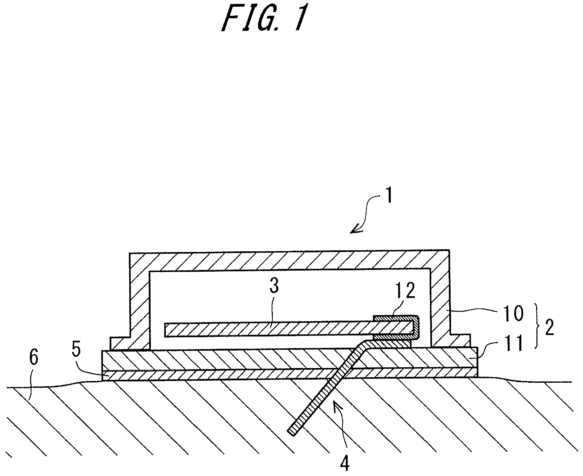

A first working example of the electrochemical sensor according to the embodiment will be described. FIG. 1 depicts a schematic view of a configuration of a composition continuous measuring apparatus 1 according to the first working example. The composition continuous measuring apparatus 1 depicted in FIG. 1 is capable of continuously measuring a concentration of a specified composition in an analyte. The analyte is exemplified by a blood and an interstitial fluid. The specified composition is exemplified such as glucose, lactic acid and bile acid. The composition continuous measuring apparatus 1 can be used in the way of being attached to a human body. The composition continuous measuring apparatus 1 includes a housing 2, a circuit board 3 and an electrochemical sensor (detecting device) 4.

The housing 2 includes a cover 10 and a base plate 11. A space defined by the cover 10 and the base plate 11 accommodates the circuit board 3. It is preferable that the housing 2 has a waterproofing property or a water resisting property. The cover 10 and the base plate 11 may involve using a material such as a metal and a polypropylene resin each exhibiting an extremely low water permeability.

The base plate 11 is a portion into which the electrochemical sensor 4 is inserted, and fixes a part of the electrochemical sensor 4. A bonding film 5 is fixed to the base plate 11. The bonding film 5 is used when fixing the composition continuous measuring apparatus 1 to a skin 6. The bonding film 5 can involve using a double-sided adhesive tape.

The circuit board 3 is mounted with electronic components required for predetermined operations (such as applying a voltage, calculating the concentration of the specified composition or performing communications with the outside) of the composition continuous measuring apparatus 1. The circuit board 3 includes a terminal 12 for establishing an electric connection with the electrochemical sensor 4. The terminal 12 is employed for acquiring a response current value from the electrochemical sensor 4 by applying the voltage to the electrochemical sensor 4.

The electrochemical sensor 4 serves to acquire a response corresponding to the concentration of the specified composition in the analyte. A part of the electrochemical sensor 4 projects from the skin 6 and contacts the terminal 12 of the circuit board 3, while another part of the electrochemical sensor 4 is inserted into the skin 6. Namely, the electrochemical sensor 4 is employed in a way that internally (subcutaneously) indwells in the skin 6.

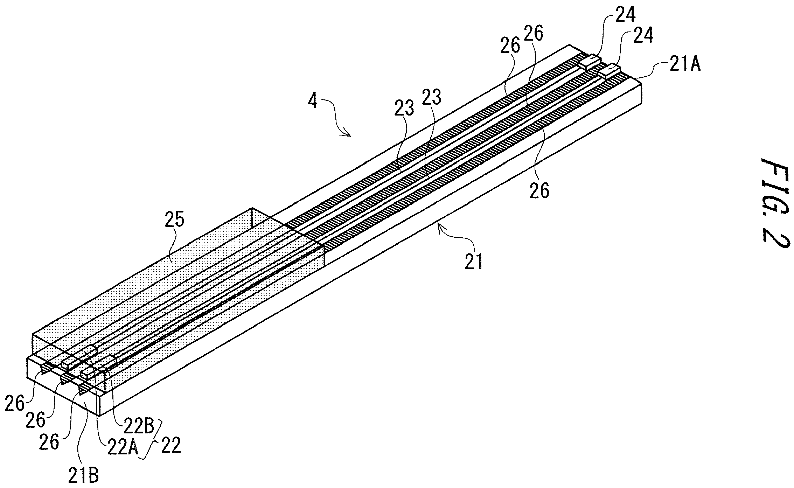

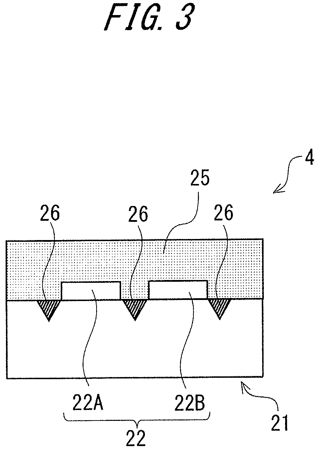

FIG. 2 is a perspective view of the whole electrochemical sensor 4 according to the first working example. FIG. 3 is a sectional view of the electrochemical sensor 4 according to the first working example. The electrochemical sensor 4 includes a substrate 21, an electrode 22, lead wires 23, a terminal 24 and an external layer film 25.

The substrate 21 has an insulating property and flexibility, and supports the electrode 22. A portion including an end portion 21A of the substrate 21 is housed inside the housing 2. A portion including an end portion 21B, opposite to the end portion 21A, of the substrate 21 is inserted into the skin 6. The end portion 21B of the substrate 21 may take an acute shape. The shape of the end portion 21B of the substrate 21 is set acute, thereby enabling the insertion of the electrochemical sensor 4 into the skin 6 to be facilitated and a pain of an examinee undergoing the insertion of the electrochemical sensor 4 to be relieved.

The substrate 21 can involve using a material exhibiting biocompatibility and the insulating property. For example, resins such as polypropylene, polyimide, polyethylene terephthalate, polyether ether ketone and polyethylene naphthalate can be used for the substrate 21. The substrate 21 is equal to or larger than, e.g., 2 mm and preferably 5 mm but equal to or smaller than 50 mm and preferably 30 mm long in a longitudinal direction. The substrate 21 is equal to or larger than, e.g., 0.05 mm and preferably 0.1 mm but equal to or smaller than 5 mm and preferably 3 mm long in a widthwise direction. The longitudinal direction of the substrate 21 is defined as a direction extending from the end portion 21B of the substrate 21 toward the end portion 21A of the substrate 21 (the direction in which the substrate 21 is housed inside the housing 2) or a direction extending from the end portion 21A of the substrate 21 toward the end portion 21B of the substrate 21 (the direction in which the substrate 21 is inserted into the skin 6). The widthwise direction of the substrate 21 is defined as a direction orthogonal to the longitudinal direction of the substrate 21.

The electrode 22 is provided on the substrate 21. Then, in the substrate 21, a plurality of grooves 26 is formed in parallel in a direction of arranging the electrode 22 of the substrate 21 (the direction in which the electrode 22 is provided on the substrate 21). The grooves 26 of the substrate 21 are formed in a way that extends to the end portion 21A of the substrate 21 from the end portion 21B of the substrate 21. In the present specification, the end portion 21B of the substrate 21 and a peripheral portion to the end portion 21B are referred to also as a leading end portion of the substrate 21. The grooves 26 of the substrate 21 are each formed in a concave shape. The concave shape includes a quadrangular shape, a semicircular shape, a semielliptical shape, a pyramid shape, etc.

A resist pattern is formed on the substrate 21 by, e.g., a photolithography technology, and the substrate 21 is etched with the resist pattern serving as a mask, whereby the grooves 26 can be formed in the substrate 21. Further, the grooves 26 can be formed in the substrate 21 also by etching based on laser beam machining.

The electrode 22 is provided adjacent to the grooves 26 on the substrate 21. The electrode 22 can be formed by, e. g. , vapor deposition, sputtering, printing (screen printing, gravure printing) or transfer printing, etc. The electrode 22 includes a working electrode 22A and a counter electrode 22B. The working electrode 22A is an electrode element for transferring and receiving electrons to and from the specified composition in the analyte. The counter electrode 22B is used for applying the voltage together with the working electrode 22A.

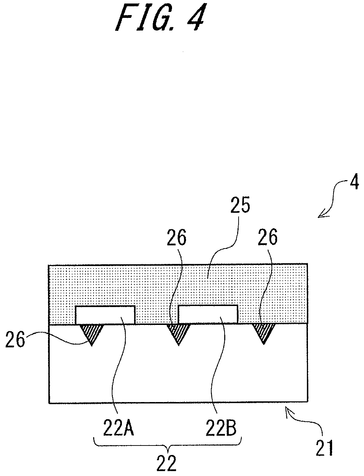

FIGS. 2 and 3 illustrate an example in which the electrode 22 is provided, on the substrate 21, adjacent to the grooves 26 of the substrate 21. In the embodiment, however, without being limited to this arrangement, the electrode 22 may be provided upwardly of the grooves 26 of the substrate 21. In this case, the electrode 22 may be provided on the substrate 21 so that at least a part of the electrode 22 is positioned upwardly of the grooves 26 of the substrate 21. FIG. 4 is a sectional view of the electrochemical sensor 4 in the case of providing the electrode 22 upwardly of the grooves 26 of the substrate 21. As depicted in FIG. 4, the working electrode 22A is provided on the substrate 21 so that a part of the working electrode 22A is positioned upwardly of the groove 26, and the counter electrode 22B is provided on the substrate 21 so that a part of the counter electrode 22B is positioned upwardly of the groove 26 of the substrate 21.

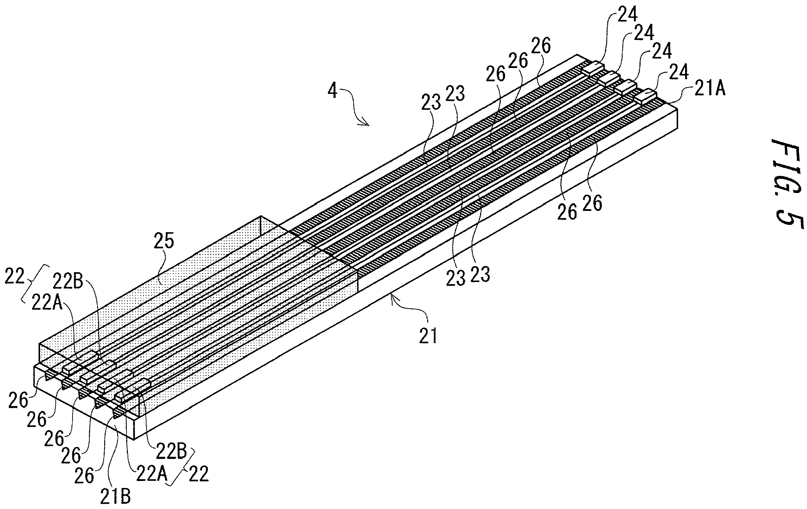

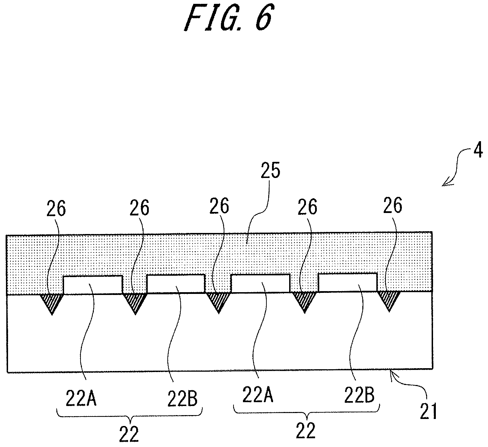

FIGS. 2, 3 and 4 each illustrate an example in which one working electrode 22A and one counter electrode 22B are provided on the substrate 21. The embodiment is not, however, limited to this configuration, and a plurality of electrodes 22 may also be provided on the substrate 21. Further, a plurality of working electrodes 22A may be provided on the substrate 21, and a plurality of counter electrodes 22B may also be provided on the substrate 21. FIG. 5 is a perspective view of the whole electrochemical sensor 4 in the case of providing the plurality of working electrodes 22A and the plurality of counter electrodes 22B on the substrate 21. FIG. 6 is a sectional view of the electrochemical sensor 4 in the case of providing the plurality of working electrodes 22A and the plurality of counter electrodes 22B on the substrate 21. In the case of providing the plurality of electrodes 22 on the substrate 21, the plurality of electrodes 22 may be provided upwardly of the grooves 26 of the substrate 21. In the case of providing the plurality of working electrodes 22A on the substrate 21, the plurality of working electrodes 22A may be provided upwardly of the grooves 26 of the substrate 21. In the case of providing the plurality of counter electrodes 22B on the substrate 21, the plurality of counter electrodes 22B may be provided upwardly of the grooves 26 of the substrate 21.

The plurality of working electrodes 22A is provided on the substrate 21, thereby making it possible to continue measuring the concentration of the specified composition in the analyte even if the malfunction such as a failure occurs in one working electrode 22A. The plurality of counter electrodes 22B is provided on the substrate 21, thereby making it possible to continue measuring the concentration of the specified composition in the analyte even if the malfunction such as the failure occurs in one counter electrode 22B. Moreover, the plurality of counter electrodes 22B is provided on the substrate 21, thereby enabling analysis target items different from each other to be measured. To be specific, the plurality of counter electrodes 22B is provided on the substrate 21, whereby plural types of specified compositions contained in the analyte can be measured.

One ends of the lead wires 23 are connected to the working electrode 22A and the counter electrode 22B, and terminals 24 are connected to the other ends of the lead wires 23. The terminal 24 is brought into contact with the terminal 12 of the circuit board 3.

The external layer film 25 is provided on the substrate 21 so as to cover the working electrode 22A, the counter electrode 22B and the grooves 26 of the substrate 21. In this case, the external layer film 25 having lower step coverage with respect to the substrate 21 is employed. Upper portions of the grooves 26 of the substrate 21 are closed by the external layer film 25 before the external layer film 25 is embedded in the grooves 26 of the substrate 21 by using the external layer film 25 exhibiting the low step coverage. Accordingly, the interior of the groove 26 of the substrate 21 is a space where the external layer film 25 does not exist. Polymer is used for the external layer film 25, in which case the step coverage of the external layer film 25 can be controlled by adjusting a concentration of polymer. In the first working example, the external layer film 25 is prevented from being embedded in the interior of the groove 26 of the substrate 21, however, without being limited to this contrivance, the external layer film 25 may be embedded in the interior of the groove 26 of the substrate 21.

As illustrated in FIGS. 2 and 5, the external layer film 25 is not provided at the leading end portion of the substrate 21, and hence some of the upper portions of the grooves 26 of the substrate 21 are not covered with the external layer film 25. Namely, the leading end portion of the substrate 21 is not provided with the external layer film 25, and the upper portions of the grooves 26 at the leading end portion of the substrate 21 are exposed.

A reagent enzyme is formed on the surface of the working electrode 22A. For instance, in the case of measuring a concentration of glucose in the analyte, the reagent enzyme can involve using glucose oxidase (GOD) or glucose dehydrogenase (GDH). Further, for example, in the case of measuring a concentration of lactate acid in the analyte, lactate oxidase can be used. A method of immobilizing the reagent enzyme can adopt a variety of known methods such as a method of utilizing MPC polymer in which a silane coupling agent is introduced into polymeric gel, high polymer such as polyacrylamide and phosphorus and phospholipid polymer, or a protein film. Moreover, the external layer film 25 may contain the reagent enzyme in place of forming the reagent enzyme on the surface of the working electrode 22A.

The external layer film 25 is structured to allow the analyte to enters inside the film itself. Namely, when the analyte contacts the external layer film 25, the analyte permeates the interior of the external layer film 25. The analyte permeating the interior of the external layer film 25 reaches the surface of the working electrode 22A. Thus, the outside of the external layer film 25 conducts to the working electrode 22A via the external layer film 25.

The material exhibiting the biocompatibility can be employed for the external layer film 25. The external layer film 25 can involve using, e.g., polyurethane, silicon-based polymer (polysiloxane), cellulose acetate, hydrogel, polyvinyl alcohol, HEMA (hydroxyethyl methacrylate), and copolymer containing these substances. The external layer film 25 can be formed by, e.g., spin coating, dip coating or drop coating, etc.

The external layer film 25 is provided on the substrate 21 in a way that covers the electrode 22. The external layer film 25 is provided so as to cover the electrode 22, and therefore, when the electrochemical sensor 4 is inserted into the skin 6, it follows that the electrode 22 does not directly contact the skin 6. Thus, the external layer film 25 functions also as a protection film for protecting the electrode 22.

When inserting the electrochemical sensor 4 into the skin 6, the analyte in the skin 6 contacts the grooves 26 of the substrate 21. At the leading end portion of the substrate 21, the upper portions of the grooves 26 of the substrate 21 are not covered with the external layer film 25, so that the analyte contacts the grooves 26 of the substrate 21 from a direction of the side surface or from a direction of the upper surface of the end portion 21B of the substrate 21.

The analyte brought into contact with the grooves 26 of the substrate 21 is introduced into the interiors of the grooves 26 of the substrate 21 by dint of a capillarity. The analyte introduced into the interiors of the grooves 26 of the substrate 21 advances through the interiors of the grooves 26 of the substrate 21 in the longitudinal direction of the substrate 21 by dint of the capillarity. The analyte advancing through the interiors of the grooves 26 of the substrate 21 contacts the lower surface of the external layer film 25 provided upwardly of the grooves 26 of the substrate 21.

The analyte coming into contact with the lower surface of the external layer film 25 permeates the interior of the external layer film 25. The analyte permeates the interior of the external layer film 25 and reaches the surface of the working electrode 22A, at which time the reagent enzyme formed on the surface of the working electrode 22A reacts on the analyte. The working electrode 22A and the counter electrode 22B apply the voltage to the reagent enzyme, thereby transferring and receiving the electrons between the specified composition contained in the analyte and the working electrode 22A. Note that if the external layer film 25 contains the reagent enzyme, the reagent enzyme contained by the external layer film 25 reacts on the analyte, and the working electrode 22A and the counter electrode 22B apply the voltage to the reagent enzyme, thereby transferring and receiving the electrons between the specified composition contained in the analyte and the working electrode 22A.

When inserting the electrochemical sensor 4 into the skin 6 and when the analyte within the skin 6 contacts the upper surface and the side surface of the external layer film 25, the analyte permeates the interior of the external layer film 25 from the upper surface and the side surface of the external layer film 25.

The external layer film 25 is provided on the substrate 21 so as to cover the working electrode 22A, and consequently the analyte permeates the interior of the external layer film 25, which leads to a state where the analyte exists in the periphery of the working electrode 22A, thus restraining depletion of the analyte on the surface of the working electrode 22A. In this way, the grooves 26 are formed in the substrate 21, the electrode 22 is provided on the substrate 21, and the external layer film 25 is provided in a manner that covers the electrode 22, thereby making it feasible to increase a quantity of supply of the analyte to the surface of the working electrode 22A. Even if only a small quantity of analyte exists in the periphery of the electrochemical sensor 4, the analyte is introduced into the interiors of the grooves 26 of the substrate 21, then advances through the interiors of the grooves 26 of the substrate 21 and is thus supplied to the surface of the working electrode 22A. For example, even when the analyte exists only at inlet ports of the grooves 26 of the substrate 21 but does not exist on the upper surface of the external layer film 25, the analyte is introduced into the interiors of the grooves 26 of the substrate 21, then advances through the interiors of the grooves 26 of the substrate 21 and is thus supplied to the surface of the working electrode 22A.

Owing to the capillarity, the analyte is introduced into the interiors of the grooves 26 of the substrate 21, then advances through the interiors of the grooves 26 of the substrate 21 and can be early supplied to the surface of the working electrode 22A. It is therefore feasible to reduce a period of time for which the surface of the working electrode 22A gets wetted with the analyte, and phenomena such as ion migration caused on the electrode surface and formation of an electric charge distribution quickly occur, thereby enabling equilibration to be done soon. As a result, it is possible to reduce a period of time of an initial operation required for stably measuring the concentration of the specified composition in the analyte.

The upper portions of the grooves 26 of the substrate 21 are covered by the external layer film 25, and hence the analyte introduced into the interiors of the grooves 26 of the substrate 21 stays within the grooves 26 of the substrate 21. Even when the quantity of the analyte in the periphery of the electrochemical sensor 4 inserted into the skin 6 decreases, if kept in the state where the analyte remains in the interiors of the grooves 26 of the substrate 21, it is feasible to restrain a risk against the depletion of the analyte on the surface of the working electrode 22A. Especially when the analyte is the interstitial fluid, since such a possibility exists that there is not the analyte quantity plentiful enough to flow in vivo, the depletion of the interstitial fluid on the surface of the working electrode 22A can be restrained by reserving the interstitial fluid in the interiors of the grooves 26 of the substrate 21.

A widthwise length and a depth of each of the grooves 26 of the substrate 21 are determined so that the analyte is introduced into the interiors of the grooves 26 of the substrate 21 by dint of the capillarity and that the analyte advances through the interiors of the grooves 26 of the substrate 21 by dint of the capillarity. The widthwise length of each of the grooves 26 of the substrate 21 is equal to or larger than, e.g., 0.05 mm but equal to or smaller than 3 mm, preferably equal to or larger than 0.1 mm but equal to or smaller than 3 mm, and more preferably equal to or larger than, e.g., 0.1 mm but equal to or smaller than 1 mm. Furthermore, the depth of each of the grooves 26 of the substrate 21 is equal to or larger than, e.g., 50 µm but equal to or smaller than 200 µm and preferably equal to or larger than 75 µm but equal to or smaller than 150 µm.

It is preferable that an internal surface of each of the grooves 26 of the substrate 21 undergoes a hydrophilization process. A variety of known methods can be adopted as a method of the hydrophilization process. The hydrophilization process may be executed by using, e.g., VUV (vacuum ultraviolet rays) process, a plasma exposure process, a surface active agent coating process, etc. Further, the hydrophilization process may also be carried out by using the same method as the method described in Japanese Unexamined Patent Publication No. 2010-532269. The internal surface of each of the grooves 26 of the substrate 21 is subjected to the hydrophilization process, thereby accelerating a degree of how much the interiors of the grooves 26 of the substrate 21 get wetted with the analyte and accelerating the advancement of the analyte through the interiors of the grooves 26 of the substrate 21. It is preferable that the hydrophilization process over the internal surfaces of the grooves 26 of the substrate 21 is performed so that a contact angle with the analyte is smaller than, e.g., 30 degrees. If the internal surfaces of the grooves 26 of the substrate 21 are hydrophilized in such an extent as to allow the analyte to advance through the interiors of the grooves 26 of the substrate 21, however, the contact angle with the analyte may be equal to or larger than 30 degrees.

It is preferable that the VUV process is conducted by use of, e.g., the ultraviolet rays (an excimer laser etc) having a wavelength of 172 nm with an intensity of 2.3 mW/cm² (in a case where a distance between the light source and the substrate 21 is 4 mm) for irradiation time that is equal to or longer than several dozens of seconds but equal to shorter than 30 minutes. The VUV process may use, without being limited to the ultraviolet rays having the wavelength of 172 nm, the ultraviolet rays having other wavelengths.

The first working example demonstrates the instance of forming the grooves 26 in the substrate 21 in a way that extends from the end portion 21B of the substrate 21 to the end portion 21A of the substrate 21. The present embodiment is not, however, limited to this formation, and the grooves 26 may be formed in the substrate 21 so as to extend at a predetermined distance from the end portion 21B of the substrate 21 in the longitudinal direction of the substrate 21. For example, the grooves 26 may also be formed in a portion of the substrate 21, which is inserted into the skin 6.

The first working example demonstrates the instance in which the upper portions of the grooves 26 of the substrate 21 are not covered with the external layer film 25 at the leading end portion of the substrate 21. The present embodiment is not, however, limited to this cover mode, and the entire upper portions of the grooves 26 of the substrate 21 may also be covered with the external layer film 25 at the leading end portion of the substrate 21. That is, the upper portions of the grooves 26 of the substrate 21 may not be exposed at the leading end portion of the substrate 21.

The first working example demonstrates the instance of forming the plurality of grooves 26 in the substrate 21. The present embodiment is not, however, limited to this formation, and only one groove 26 may also be formed in the substrate 21. In this case, the working electrode 22A and the counter electrode 22B may be provided, on the substrate 21, adjacent to the groove 26 of the substrate 21 so that the groove 26 of the substrate 21 is positioned between the working electrode 22A and the counter electrode 22B.

### <First Modified Example>

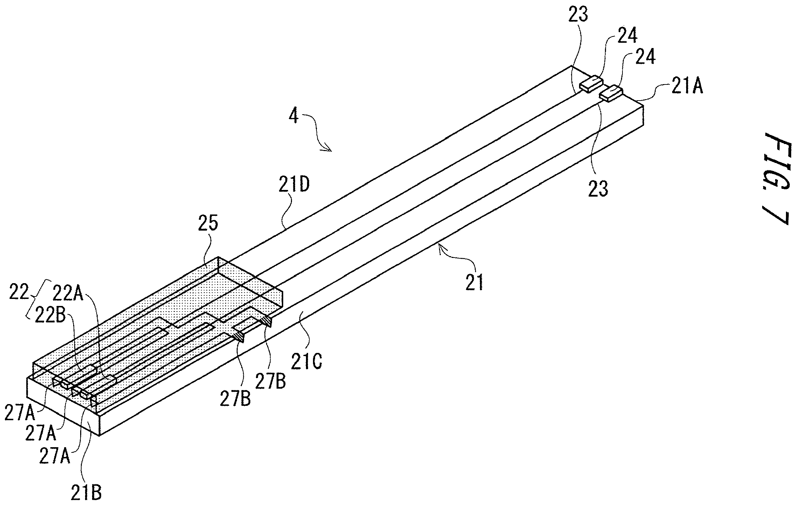

A first modified example of the first working example will be described. FIG. 7 is a perspective view of the whole electrochemical sensor 4 according to the first modified example of the first working example. The electrode 22 is provided on the substrate 21. The substrate 21 is formed with plural lines of grooves 27A and plural lines of grooves 27B. The grooves 27A of the substrate 21 are formed in the direction of arranging the electrode 22 of the substrate 21 (the direction of providing the electrode 22 on the substrate 21). The direction in which the electrode 22 is provided on the substrate 21 is coincident with the longitudinal direction of the substrate 21. The grooves 27B of the substrate 21 are formed in the widthwise direction of the substrate 21. The grooves 27B of the substrate 21 include a groove formed extending from an end portion 21C of the substrate 21 to the groove 27A of the substrate 21, and a groove which connects the grooves 27A of the substrate 21 to each other.

FIG. 7 depicts an example of providing the single working electrode 22A and the single counter electrode 22B on the substrate 21. The present embodiment is not, however, limited to this arrangement, and the plurality of electrodes 22 may also be provided on the substrate 21. Further, the plurality of working electrodes 22A may be provided on the substrate 21, and the plurality of counter electrodes 22B may also be provided on the substrate 21. A position in which the groove 27B is formed extending from the end portion 21C of the substrate 21 to the groove 27A of the substrate 21 may be an arbitrary position if being the position of a portion inserted into the skin 6. Furthermore, the grooves 27B may be formed so as to extend from an end portion 21D of the substrate 21 to the groove 27A of the substrate 21.

The grooves 27A of the substrate 21 are formed up to the near side of the end portion 21B of the substrate 21. In the first modified example of the first working example, the electrode 22 is provided on the near side of the end portion 21B of the substrate 21, and therefore the grooves 27A of the substrate 21 are formed up to the near side of the end portion 21B of the substrate 21. The present embodiment is not, however, limited to this formation, and the grooves 27A of the substrate 21 may be formed in the vicinity of the electrode 22 on the substrate 21. That is, the positions in which to form the grooves 27A in the substrate 21 are changed corresponding to the position of the electrode 22 on the substrate 21.

The external layer film 25 is provided on the substrate 21 so as to cover the working electrode 22A, the counter electrode 22B, the grooves 27A of the substrate 21 and the grooves 27B of the substrate 21. The contrivance in the first modified example of the first working example is that the external layer film 25 is embedded in neither the interior of the groove 27A of the substrate 21 nor the interior of the groove 27B of the substrate 21, however, without being confined to this contrivance, the external layer film 25 may be embedded in the interior of the groove 27A of the substrate 21 and in the interior of the groove 27B of the substrate 21.

As illustrated in FIG. 7, there is a portion where the upper portion of the groove 27B of the substrate 21 is not covered by the external layer film 25. Namely, there is exposed the upper portion of the groove 27B at the peripheral portion of the end portion 21C of the substrate 21. In the case of inserting the electrochemical sensor 4 into the skin 6, the analyte within the skin 6 contacts the groove 27B of the substrate 21. The upper portion of the groove 27B of the substrate 21 is not covered by the external layer film 25 at the peripheral portion of the end portion 21C of the substrate 21, and hence the analyte contacts the groove 27B of the substrate 21 from the direction of the side surface or the upper surface of the substrate 21.

The analyte coming into contact with the grooves 27B of the substrate 21 is introduced into the interiors of the grooves 27B of the substrate 21 by dint of the capillarity. The analyte introduced into the interiors of the grooves 27B of the substrate 21 advances through the interiors of the grooves 27B of the substrate 21 by dint of the capillarity in the widthwise direction of the substrate 21. Then, the analyte contacts the lower surface of the external layer film 25 provided upwardly of the grooves 27B of the substrate 21. The analyte contacting the lower surface of the external layer film 25 permeates the interior of the external layer film 25 and is thus supplied to the surface of the working electrode 22A.

Further, the analyte advancing through the interiors of the grooves 27B of the substrate 21 in the widthwise direction of the substrate 21 is introduced into the interiors of the grooves 27A of the substrate 21. The analyte introduced into the interiors of the grooves 27A of the substrate 21 advances through the interiors of the grooves 27A of the substrate 21 by dint of the capillarity in the longitudinal direction of the substrate 21. Then, the analyte advances through the interiors of the grooves 27A of the substrate 21 up to the near side of the end portion 21B of the substrate 21, and comes into contact with the lower surface of the external layer film 25 provided upwardly of the grooves 27A of the substrate 21. The analyte coming into contact with the lower surface of the external layer film 25 permeates the interior of the external layer film 25 and is thus supplied to the surface of the working electrode 22A.

Even if only the small quantity of analyte exists in the periphery of the electrochemical sensor 4, the analyte advances through the interiors of the grooves 27A of the substrate 21 and the interiors of the grooves 27B of the substrate 21 and is thus supplied to the surface of the working electrode 22A. For example, even when the analyte exists only in the vicinity of inlet ports of the grooves 27B of the substrate 21 but does not exist on the upper surface of the external layer film 25, the analyte advances through the interiors of the grooves 27A of the substrate 21 and the interiors of the grooves 27B of the substrate 21 and is thus supplied to the surface of the working electrode 22A.

A widthwise length and a depth of each of the grooves 27A of the substrate 21 are determined so that the analyte is introduced into the interiors of the grooves 27A of the substrate 21 by dint of the capillarity and that the analyte advances through the interiors of the grooves 27A of the substrate 21 by dint of the capillarity. The widthwise length of each of the grooves 27A of the substrate 21 is equal to or larger than, e.g., 0.05 mm but equal to or smaller than 3 mm, preferably equal to or larger than 0.1 mm but equal to or smaller than 3 mm, and more preferably equal to or larger than, e.g., 0.1 mm but equal to or smaller than 1 mm. Further, the depth of each of the grooves 27A of the substrate 21 is equal to or larger than, e.g., 50 µm but equal to or smaller than 200 µm and preferably equal to or larger than 75 µm but equal to or smaller than 150 µm. Furthermore, a widthwise length and a depth of each of the grooves 27B of the substrate 21 are determined so that the analyte is introduced into the interiors of the grooves 27B of the substrate 21 by dint of the capillarity and that the analyte advances through the interiors of the grooves 27B of the substrate 21 by dint of the capillarity. The widthwise length of each of the grooves 27B of the substrate 21 is equal to or larger than, e.g., 0.05 mm but equal to or smaller than 3 mm, preferably equal to or larger than 0.1 mm but equal to or smaller than 3 mm, and more preferably equal to or larger than, e.g., 0.1 mm but equal to or smaller than 1 mm. Further, the depth of each of the grooves 27B of the substrate 21 is equal to or larger than, e.g., 50 µm but equal to or smaller than 200 µm and preferably equal to or larger than 75 µm but equal to or smaller than 150 µm.

It is preferable that an internal surface of each of the grooves 27A and 27B of the substrate 21 undergoes the hydrophilization process. A variety of known methods can be adopted as a method of the hydrophilization process. The hydrophilization process may be executed by using, e.g., the VUV (vacuum ultraviolet rays) process, the plasma exposure process, the surface active agent coating process, etc. Further, the hydrophilization process may also be carried out by using the same method as the method described in Japanese Unexamined Patent Publication No. 2010-532269. The internal surface of each of the grooves 27A and 27B of the substrate 21 is subjected to the hydrophilization process, thereby accelerating a degree of how much the interiors of the grooves 27A and 27B of the substrate 21 get wetted with the analyte and accelerating the advancement of the analyte through the interiors of the grooves 27A and 27B of the substrate 21. It is preferable that the hydrophilization process over the internal surfaces of the grooves 27A and 27B of the substrate 21 is performed so that a contact angle with the analyte is smaller than, e.g., 30 degrees. If the internal surfaces of the grooves 27A and 27B of the substrate 21 are hydrophilized in such an extent as to allow the analyte to advance through the interiors of the grooves 27A and 27B of the substrate 21, however, the contact angle with the analyte may be equal to or larger than 30 degrees.

It is preferable that the VUV process is conducted by use of, e.g., the ultraviolet rays (the excimer laser etc) having the wavelength of 172 nm with the intensity of 2. 3 mW/cm² (in a case where a distance between the light source and the substrate 21 is 4 mm) for irradiation time that is equal to or longer than several dozens of seconds but equal to shorter than 30 minutes. The VUV process may use, without being limited to the ultraviolet rays having the wavelength of 172 nm, the ultraviolet rays having other wavelengths.

The first modified example of the first working example demonstrates the instance in which the upper portion of the substrate 21 is not covered by the external layer film 25 at the peripheral portion of the end portion 21C of the substrate 21. The present embodiment is not, however, limited to this cover mode, and the entire upper portion of the groove 27B of the substrate 21 may be covered by the external layer film 25 at the peripheral portion of the end portion 21C of the substrate 21. That is, the upper portion of the groove 27B of the substrate 21 may not be exposed at the peripheral portion of the end portion 21C of the substrate 21.

The first modified example of the first working example demonstrates the instance in which the electrode 22 is provided, on the substrate 21, adjacent to the grooves 27A of the substrate 21. The present embodiment is not, however, limited to this configuration, and the electrode 22 may be provided upwardly of the groove 27A of the substrate 21. In this case, the electrode 22 may be provided on the substrate 21 so that at least a part of the electrode 22 is positioned upwardly of the groove 27A of the substrate 21.

The first modified example of the first working example demonstrates the instance in which the plural lines of grooves 27A are formed in the substrate 21. The present embodiment is not, however, limited to this formation, and only one line of groove 27A may also be formed in the substrate 21. In this case, the groove 27A of the substrate 21 may be positioned between the working electrode 22A and the counter electrode 22B. Further, the first modified example of the first working example demonstrates the instance in which the plural lines of grooves 27B are formed in the substrate 21. The present embodiment is not, however, limited to this formation, and only one line of groove 27B may also be formed in the substrate 21.

### <Second Modified Example>

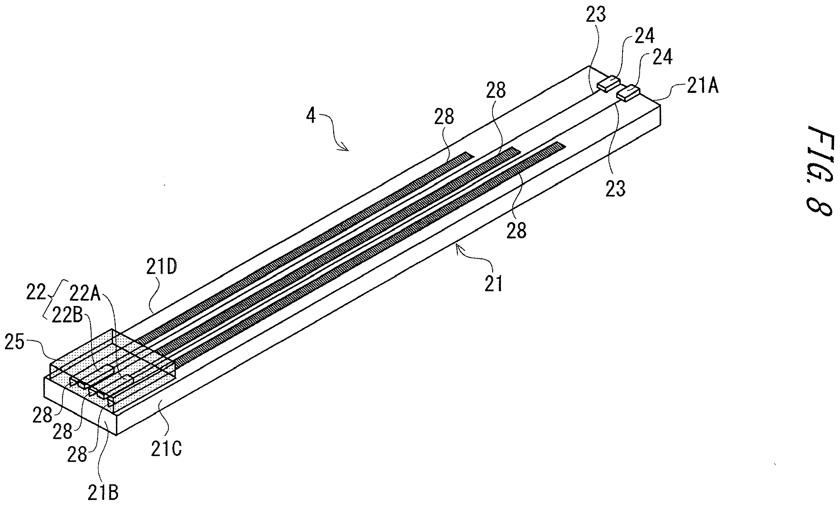

A second modified example of the first working example will be described. FIG. 8 is a perspective view of the whole electrochemical sensor 4 according to the second modified example of the first working example. The electrode 22 is provided on the substrate 21. The substrate 21 is formed with plural lines of grooves 28. The grooves 28 of the substrate 21 are formed in the direction of arranging the electrode 22 of the substrate 21 (the direction of providing the electrode 22 on the substrate 21). The direction in which the electrode 22 is provided on the substrate 21 is coincident with the longitudinal direction of the substrate 21.

The grooves 28 of the substrate 21 are formed up to the near side of the end portion 21B of the substrate 21. The external layer film 25 is provided on the substrate 21 so as to cover the working electrode 22A, the counter electrode 22B and a part of the grooves 28 of the substrate 21. The contrivance in the second modified example of the first working example is that the external layer film 25 is not embedded in the interiors of the grooves 28 of the substrate 21, however, without being confined to this contrivance, the external layer film 25 may be embedded in the interiors of the grooves 28 of the substrate 21.

As depicted in FIG. 8, there is a portion where the upper portion of the groove 28 of the substrate 21 is not covered by the external layer film 25. Namely, the upper portion of a part of the groove 28 of the substrate 21 is exposed. In the case of inserting the electrochemical sensor 4 into the skin 6, the analyte within the skin 6 contacts the part of the groove 28 of the substrate 21. The upper portion of the part of the groove 28 of the substrate 21 is not covered by the external layer film 25, and therefore the analyte contacts the groove 28 of the substrate 21 from the direction of the upper surface of the substrate 21.

The analyte coming into contact with the grooves 28 of the substrate 21 is introduced into the interiors of the grooves 28 of the substrate 21 by dint of the capillarity. The analyte introduced into the interiors of the grooves 28 of the substrate 21 advances through the interiors of the grooves 28 of the substrate 21 by dint of the capillarity in the longitudinal direction of the substrate 21. The analyte advancing through the interiors of the grooves 28 of the substrate 21 in the longitudinal direction of the substrate 21 contacts the lower surface of the external layer film 25 provided upwardly of the grooves 28 of the substrate 21. The analyte contacting the lower surface of the external layer film 25 permeates the interior of the external layer film 25 and is thus supplied to the surface of the working electrode 22A.

Even if only the small quantity of analyte exists in the periphery of the electrochemical sensor 4, the analyte is introduced into the interiors of the grooves 28 of the substrate 21 and is thereby supplied to the surface of the working electrode 22A. For example, the analyte does not exist in the periphery of the external layer film 25 but exists only in the portion where the upper portions of the grooves 28 of the substrate 21 are not covered by the external layer film 25, even in which case the analyte is introduced into the interiors of the grooves 28 of the substrate 21 and is thereby supplied to the surface of the working electrode 22A.

A widthwise length and a depth of each of the grooves 28 of the substrate 21 are determined so that the analyte is introduced into the interiors of the grooves 28 of the substrate 21 by dint of the capillarity and that the analyte advances through the interiors of the grooves 28 of the substrate 21 by dint of the capillarity. The widthwise length of each of the grooves 28 of the substrate 21 is equal to or larger than, e.g., 0.05 mm but equal to or smaller than 3 mm, preferably equal to or larger than 0.1 mm but equal to or smaller than 3 mm, and more preferably equal to or larger than, e.g., 0.1 mm but equal to or smaller than 1 mm. Further, the depth of each of the grooves 28 of the substrate 21 is equal to or larger than, e.g., 50 µm but equal to or smaller than 200 µm and preferably equal to or larger than 75 µm but equal to or smaller than 150 µm.

It is preferable that an internal surface of each of the grooves 28 of the substrate 21 undergoes the hydrophilization process. A variety of known methods can be adopted as a method of the hydrophilization process. The hydrophilization process may be executed by using, e.g., the VUV (vacuum ultraviolet rays) process, the plasma exposure process, the surface active agent coating process, etc. Further, the hydrophilization process may also be carried out by using the same method as the method described in Japanese Unexamined Patent Publication No. 2010-532269. The internal surface of each of the grooves 28 of the substrate 21 is subjected to the hydrophilization process, thereby accelerating a degree of how much the interiors of the grooves 28 of the substrate 21 get wetted with the analyte and accelerating the advancement of the analyte through the interiors of the grooves 28 of the substrate 21. It is preferable that the hydrophilization process over the internal surfaces of the grooves 28 of the substrate 21 is performed so that a contact angle with the analyte is smaller than, e.g., 30 degrees. If the internal surfaces of the grooves 28 of the substrate 21 are hydrophilized in such an extent as to allow the analyte to advance through the interiors of the grooves 28 of the substrate 21, however, the contact angle with the analyte may be equal to or larger than 30 degrees.

It is preferable that the VUV process is conducted by use of, e.g., the ultraviolet rays (the excimer laser etc) having the wavelength of 172 nm with the intensity of 2.3 mW/cm² (in a case where a distance between the light source and the substrate 21 is 4 mm) for irradiation time that is equal to or longer than several dozens of seconds but equal to shorter than 30 minutes. The VUV process may use, without being limited to the ultraviolet rays having the wavelength of 172 nm, the ultraviolet rays having other wavelengths.

The second modified example of the first working example demonstrates the instance in which the electrode 22 is provided, on the substrate 21, adjacent to the grooves 28 of the substrate 21. The present embodiment is not, however, limited to this configuration, and the electrode 22 may be provided upwardly of the groove 28 of the substrate 21. In this case, the electrode 22 may be provided on the substrate 21 so that at least a part of the electrode 22 is positioned upwardly of the groove 28 of the substrate 21.

The second modified example of the first working example demonstrates the instance in which the plural lines of grooves 28 are formed in the substrate 21. The present embodiment is not, however, limited to this formation, and only one line of groove 28 may also be formed in the substrate 21. In this case, the groove 28 of the substrate 21 may be positioned between the working electrode 22A and the counter electrode 22B.

### <Third Modified Example>

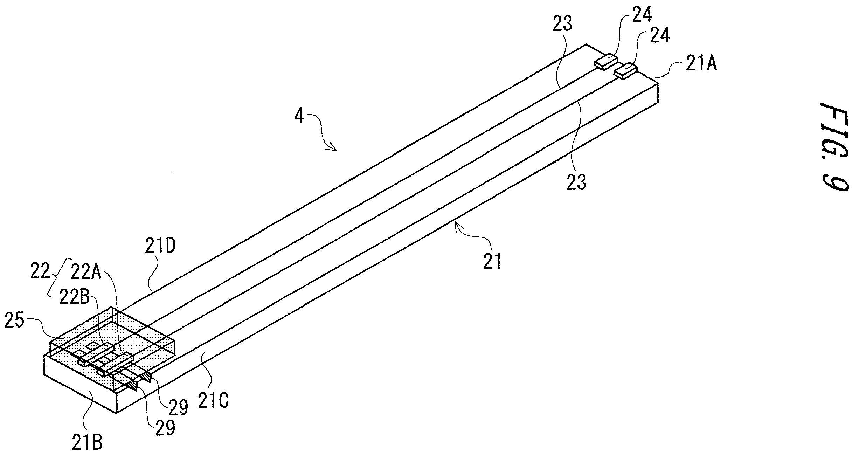

A third modified example of the first working example will be described. FIG. 9 is a perspective view of the whole electrochemical sensor 4 according to the third modified example of the first working example. The electrode 22 is provided on the substrate 21. The substrate 21 is formed with plural lines of grooves 29. The grooves 29 of the substrate 21 are formed in the direction of arranging the electrode 22 of the substrate 21 (the direction of providing the electrode 22 on the substrate 21). The direction in which the electrode 22 is provided on the substrate 21 is coincident with the widthwise direction of the substrate 21.

The groove 29 of the substrate 21 is formed in the substrate 21 in a way that intersects the electrode 22 on the substrate 21. In this case, the electrode 22 is provided on the substrate 21 so that a part of the electrode 22 is positioned upwardly of the groove 29 of the substrate 21. To be specific, the working electrode 22A is provided on the substrate 21 so that a part of the working electrode 22A is positioned upwardly of the groove 29 of the substrate 21, and the counter electrode 22B is provided on the substrate 21 so that a part of the counter electrode 22B is positioned upwardly of the groove 29 of the substrate 21.

FIG. 9 depicts an example of providing the single working electrode 22A and the single counter electrode 22B on the substrate 21. The present embodiment is not, however, limited to this arrangement, and the plurality of electrodes 22 may also be provided on the substrate 21. Further, the plurality of working electrodes 22A may be provided on the substrate 21, and the plurality of counter electrodes 22B may also be provided on the substrate 21.

The external layer film 25 is provided on the substrate 21 so as to cover the working electrode 22A, the counter electrode 22B and the grooves 29 of the substrate 21. The contrivance in the third modified example of the first working example is that the external layer film 25 is not embedded in the interior of the groove 29 of the substrate 21, however, without being confined to this contrivance, the external layer film 25 may be embedded in the interior of the groove 29 of the substrate 21.

As depicted in FIG. 9, there is a portion where the upper portion of the groove 29 of the substrate 21 is not covered by the external layer film 25. Namely, the upper portion of the groove 29 at the peripheral portion of the end portion 21C of the substrate 21 is exposed. In the case of inserting the electrochemical sensor 4 into the skin 6, the analyte within the skin 6 contacts the groove 29 of the substrate 21. At the peripheral portion of the end portion 21C of the substrate 21, the upper portion of the groove 29 of the substrate 21 is not covered by the external layer film 25, and therefore the analyte contacts the groove 29 of the substrate 21 from the direction of the side surface or the upper surface of the substrate 21.

The analyte coming into contact with the grooves 29 of the substrate 21 is introduced into the interiors of the grooves 29 of the substrate 21 by dint of the capillarity. The analyte introduced into the interiors of the grooves 29 of the substrate 21 advances through the interiors of the grooves 29 of the substrate 21 by dint of the capillarity in the widthwise direction of the substrate 21. Then, the analyte contacts the lower surface of the external layer film 25 provided upwardly of the grooves 29 of the substrate 21. The analyte contacting the lower surface of the external layer film 25 permeates the interior of the external layer film 25 and is thus supplied to the surface of the working electrode 22A.

Furthermore, the analyte advancing through the interiors of the grooves 29 of the substrate 21 in the widthwise direction of the substrate 21 comes into contact with the working electrode 22A provided upwardly of the grooves 29 of the substrate 21 and is thus supplied to the surface of the working electrode 22A.

Even if only the small quantity of analyte exists in the periphery of the electrochemical sensor 4, the analyte advances through the interiors of the grooves 29 of the substrate 21 and is thereby supplied to the surface of the working electrode 22A. For example, even when the analyte exists only in the vicinity of inlet ports of the grooves 29 of the substrate 21 but does not exist on the upper surface of the external layer film 25, the analyte advances through the interiors of the grooves 29 of the substrate 21 and is thus supplied to the surface of the working electrode 22A.

A widthwise length and a depth of each of the grooves 29 of the substrate 21 are determined so that the analyte is introduced into the interiors of the grooves 29 of the substrate 21 by dint of the capillarity and that the analyte advances through the interiors of the grooves 29 of the substrate 21 by dint of the capillarity. The widthwise length of each of the grooves 29 of the substrate 21 is equal to or larger than, e.g. , 0.05 mm but equal to or smaller than 3 mm, preferably equal to or larger than 0.1 mm but equal to or smaller than 3 mm, and more preferably equal to or larger than, e.g., 0.1 mm but equal to or smaller than 1 mm. Further, the depth of each of the grooves 29 of the substrate 21 is equal to or larger than, e.g., 50 µm but equal to or smaller than 200 µm and preferably equal to or larger than 75 µm but equal to or smaller than 150 µm.

It is preferable that an internal surface of each of the grooves 29 of the substrate 21 undergoes the hydrophilization process. A variety of known methods can be adopted as a method of the hydrophilization process. The hydrophilization process may be executed by using, e.g., the VUV (vacuum ultraviolet rays) process, the plasma exposure process, the surface active agent coating process, etc. Further, the hydrophilization process may also be carried out by using the same method as the method described in Japanese Unexamined Patent Publication No. 2010-532269. The internal surface of each of the grooves 29 of the substrate 21 is subjected to the hydrophilization process, thereby accelerating a degree of how much the interiors of the grooves 29 of the substrate 21 get wetted with the analyte and accelerating the advancement of the analyte through the interiors of the grooves 29 of the substrate 21. It is preferable that the hydrophilization process over the internal surfaces of the grooves 29 of the substrate 21 is performed so that a contact angle with the analyte is smaller than, e.g., 30 degrees. If the internal surfaces of the grooves 29 of the substrate 21 are hydrophilized in such an extent as to allow the analyte to advance through the interiors of the grooves 29 of the substrate 21, however, the contact angle with the analyte may be equal to or larger than 30 degrees.

It is preferable that the VUV process is conducted by use of, e.g., the ultraviolet rays (the excimer laser etc) having the wavelength of 172 nm with the intensity of 2. 3 mW/cm² (in a case where a distance between the light source and the substrate 21 is 4 mm) for irradiation time that is equal to or longer than several dozens of seconds but equal to shorter than 30 minutes. The VUV process may use, without being limited to the ultraviolet rays having the wavelength of 172 nm, the ultraviolet rays having other wavelengths.

The third modified example of the first working example demonstrates the instance in which the upper portion of the substrate 21 is not covered by the external layer film 25 at the peripheral portion of the end portion 21C of the substrate 21. The present embodiment is not, however, limited to this cover mode, and the entire upper portion of the groove 29 of the substrate 21 may be covered by the external layer film 25 at the peripheral portion of the end portion 21C of the substrate 21. That is, the upper portion of the groove 29 of the substrate 21 may not be exposed at the peripheral portion of the end portion 21C of the substrate 21.

The third modified example of the first working example demonstrates the instance in which the electrode 22 is provided, on the substrate 21, adjacent to the grooves 29 of the substrate 21. The present embodiment is not, however, limited to this configuration, and the electrode 22 may be provided upwardly of the groove 29 of the substrate 21. In this case, the electrode 22 may be provided on the substrate 21 so that at least a part of the electrode 22 is positioned upwardly of the groove 29 of the substrate 21.

The third modified example of the first working example demonstrates the instance of forming the plural lines of grooves 29 in the substrate 21. The present embodiment is not, however, limited to this formation, and only one line of groove 29 may be formed in the substrate 21.

### Second Working Example

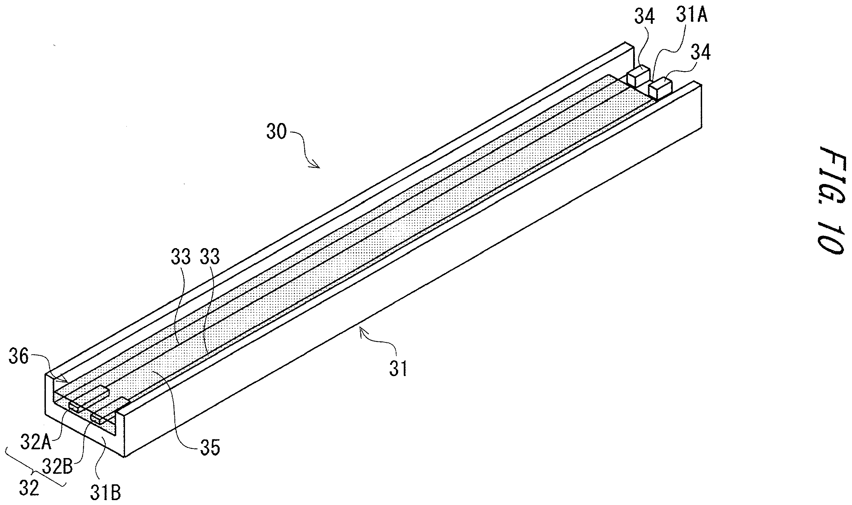

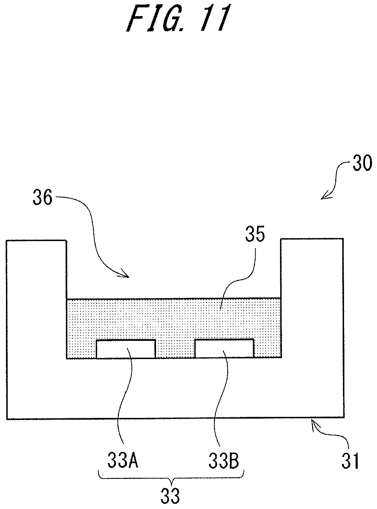

A second working example of the electrochemical sensor according to the embodiment will be described. FIG. 10 is a perspective view of a whole electrochemical sensor 30 according to the second working example. FIG. 11 is a sectional view of the electrochemical sensor 30 according to the second working example. Similarly to the first working example, the electrochemical sensor 30 is inserted into the base plate 11 of the composition continuous measuring apparatus 1 illustrated in FIG. 1, and the end portion of the electrochemical sensor 30 is fixed to the base plate 11. The electrochemical sensor 30 includes a substrate 31, an electrode 32, lead wires 33, a terminal 34 and an external layer film 35.

The substrate 31 has an insulating property and flexibility, and supports the electrode 32. A portion including an end portion 31A of the substrate 31 is housed inside the housing 2. A portion including an end portion 31B, opposite to the end portion 31A, of the substrate 31 is inserted into the skin 6. The end portion 31B of the substrate 31 may take an acute shape. The shape of the end portion 31B of the substrate 31 is set acute, thereby enabling the insertion of the electrochemical sensor 30 into the skin 6 to be facilitated and a pain of an examinee undergoing the insertion of the electrochemical sensor 30 to be relieved.

The substrate 31 can involve using a material exhibiting biocompatibility and the insulating property. For example, resins such as polypropylene, polyimide, polyethylene terephthalate, polyether ether ketone and polyethylene naphthalate can be used for the substrate 31. The substrate 31 is equal to or larger than, e.g., 2 mm and preferably 5 mm but equal to or smaller than 50 mm and preferably 30 mm long in a longitudinal direction. The substrate 31 is equal to or larger than, e.g., 0.05 mm and preferably 0.1 mm but equal to or smaller than 5 mm and preferably 3 mm long in a widthwise direction. The longitudinal direction of the substrate 31 is defined as a direction extending from the end portion 31B of the substrate 31 toward the end portion 31A of the substrate 31 (the direction in which the substrate 31 is housed inside the housing 2) or a direction extending from the end portion 31A of the substrate 31 toward the end portion 31B of the substrate 31 (the direction in which the substrate 31 is inserted into the skin 6). The widthwise direction of the substrate 31 is defined as a direction orthogonal to the longitudinal direction of the substrate 31.

The substrate 31 is formed with a groove 36 extending in the longitudinal direction of the substrate 31. The groove 36 of the substrate 31 is formed extending from an end portion 31B of the substrate 31 to an end portion 31A of the substrate 31. In the present specification, the end portion 31B of the substrate 31 and a peripheral portion to the end portion 31B are referred to also as a leading end portion of the substrate 31. The groove 36 of the substrate 31 is formed in a concave shape. The concave shape includes a quadrangular shape, a semicircular shape, a semielliptical shape, a pyramid shape, etc.

A resist pattern is formed on the substrate 31 by, e.g., a photolithography technology, and the substrate 31 is etched with the resist pattern serving as a mask, whereby the groove 36 can be formed in the substrate 31. Further, the groove 36 can be formed in the substrate 31 also by etching based on laser beam machining.

An interior of the groove 36 of the substrate 31 is provided with the electrode 32, the lead wires 33, the terminal 34 and the external layer film 35. The electrode 32 can be formed by, e.g., vapor deposition, sputtering, printing (screen printing, gravure printing) or transfer printing, etc. The electrode 32 includes a working electrode 32A and a counter electrode 32B. The working electrode 32A is an electrode element for transferring and receiving electrons to and from the specified composition in the analyte. The counter electrode 32B is used for applying the voltage together with the working electrode 32A.

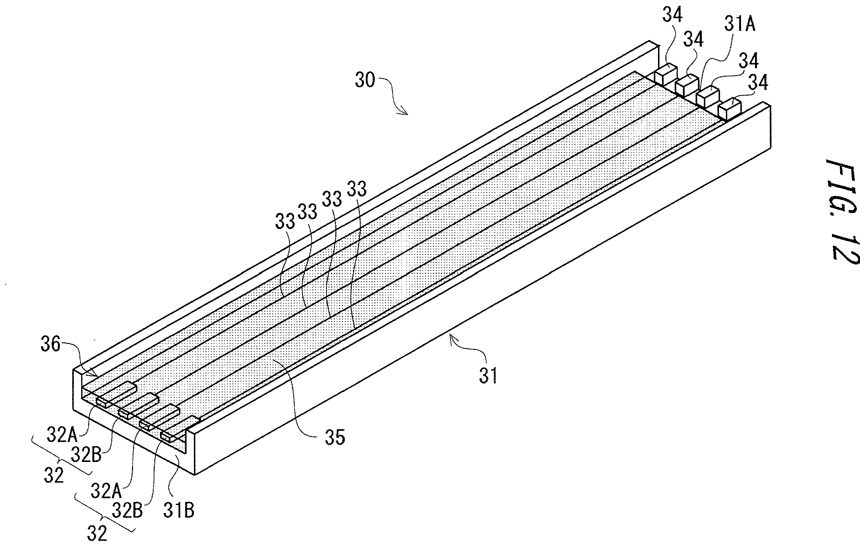

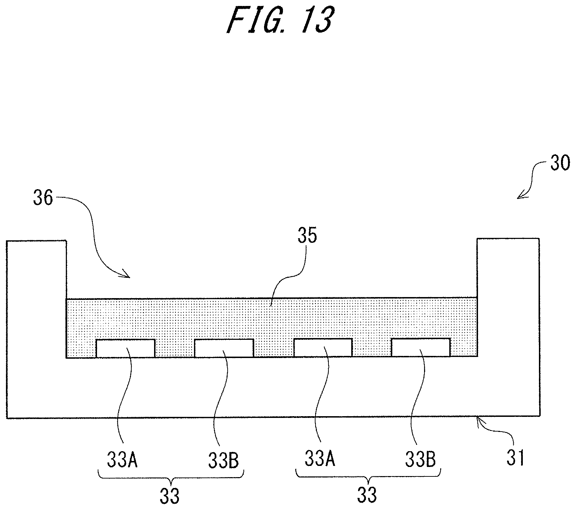

FIGS. 10 and 11 illustrate an example in which a single working electrode 32A and a single counter electrode 32B are provided in the interior of the groove 36 of the substrate 31. The present embodiment is not, however, limited to this configuration, and a plurality of electrodes 32 may be provided in the interior of the groove 36 of the substrate 31. Further, a plurality of working electrodes 32A may be provided in the interior of the groove 36 of the substrate 31, and a plurality of counter electrodes 32B may also be provided in the interior of the groove 36 of the substrate 31. FIG. 12 is a perspective view of the whole electrochemical sensor 30 in the case of providing the plurality of working electrodes 32A and the plurality of counter electrodes 32B in the interior of the groove 36 of the substrate 31. FIG. 13 is a sectional view in the case of providing the plurality of working electrodes 32A and the plurality of counter electrodes 32B in the interior of the groove 36 of the substrate 31.

The plurality of working electrodes 32A is provided on the substrate 31, thereby making it possible to continue measuring the concentration of the specified composition in the analyte even if the malfunction such as a failure occurs in one working electrode 32A. The plurality of counter electrodes 32B is provided on the substrate 31, thereby making it possible to continue measuring the concentration of the specified composition in the analyte even if the malfunction such as the failure occurs in one counter electrode 32B.

One ends of the lead wires 33 are connected to the working electrode 32A and the counter electrode 32B, and terminals 34 are connected to the other ends of the lead wires 33. The terminal 34 is brought into contact with the terminal 12 of the circuit board 3.

A reagent enzyme is formed on the surface of the working electrode 32A. For instance, in the case of measuring a concentration of glucose in the analyte, the reagent enzyme can involve using glucose oxidase (GOD) or glucose dehydrogenase (GDH). Further, for example, in the case of measuring a concentration of lactate acid in the analyte, lactate oxidase can be used. A method of immobilizing the reagent enzyme can adopt a variety of known methods such as a method of utilizing MPC polymer in which a silane coupling agent is introduced into polymeric gel, high polymer such as polyacrylamide and phosphorus and phospholipid polymer, or a protein film. Moreover, the external layer film 35 may contain the reagent enzyme in place of forming the reagent enzyme on the surface of the working electrode 32A.

The external layer film 35 is structured to allow the analyte to enters inside the film itself. Namely, when the analyte contacts the external layer film 35, the analyte permeates the interior of the external layer film 35. The analyte permeating the interior of the external layer film 35 reaches the surface of the working electrode 32A. Thus, the outside of the external layer film 35 conducts to the working electrode 32A via the external layer film 35.

The material exhibiting the biocompatibility can be employed for the external layer film 35. The external layer film 35 can involve using, e.g., polyurethane, silicon-based polymer (polysiloxane), cellulose acetate, hydrogel, polyvinyl alcohol, HEMA (hydroxyethyl methacrylate), and copolymer containing these substances. The external layer film 35 can be formed by, e.g., spin coating, dip coating or drop coating, etc.

The external layer film 35 is provided in the interior of the groove 36 of the substrate 31 so as to cover the electrode 32. The external layer film 35 is provided so as to cover the electrode 32, and therefore, when the electrochemical sensor 30 is inserted into the skin 6, it follows that the electrode 32 does not directly contact the skin 6. Thus, the external layer film 35 functions also as a protection film for protecting the electrode 32.

In the case of inserting the electrochemical sensor 30 into the skin 6, the analyte within the skin 6 contacts the groove 36 of the substrate 31. The analyte coming into contact with the groove 36 of the substrate 31 is introduced into the interior of the groove 36 of the substrate 31 by dint of the capillarity. The analyte introduced into the interior of the groove 36 of the substrate 31 permeates the interior of the external layer film 35.

The analyte permeates the interior of the external layer film 35 and reaches the surface of the working electrode 32A, at which time the reagent enzyme formed on the surface of the working electrode 32A reacts on the analyte. The working electrode 32A and the counter electrode 32B apply the voltage to the reagent enzyme, thereby transferring and receiving the electrons between the specified composition contained in the analyte and the working electrode 32A. Note that if the external layer film 35 contains the reagent enzyme, the reagent enzyme contained by the external layer film 35 reacts on the analyte, and the working electrode 32A and the counter electrode 32B apply the voltage to the reagent enzyme, thereby transferring and receiving the electrons between the specified composition contained in the analyte and the working electrode 32A.

The electrode 32 and the external layer film 35 are formed in the interior of the groove 36 of the substrate 31. The analyte is introduced into the interior of the groove 36 of the substrate 31 by dint of the capillarity. The analyte introduced into the interior of the groove 36 of the substrate 31 contacts the upper surface of the external layer film 35. The analyte brought into contact with the upper surface of the external layer film 35 permeates the interior of the external layer film 35. Since the external layer film 35 is provided in the interior of the groove 36 of the substrate 31 so as to cover the working electrode 32A, the analyte permeating the interior of the external layer film 35 comes to a state of existing in the periphery of the working electrode 32A, and consequently the deletion of the analyte on the surface of the working electrode 32A is restrained. Thus, the substrate 31 is formed with the groove 36, and the electrode 32 and the external layer film 35 are provided in the interior of the groove 36 of the substrate 31, thereby making it feasible to increase a quantity of supply of the analyte to the surface of the working electrode 32A.

Because of the analyte being introduced into the interior of the groove 36 of the substrate 31 by dint of the capillarity, the analyte can be early supplied to the surface of the working electrode 32A, and it is possible to reduce the period of time till the surface of the working electrode 32A gets wetted with the analyte. As a result, it is possible to reduce the period of time of the initial operation required for stably measuring the concentration of the specified composition in the analyte.

The analyte introduced into the interior of the groove 36 of the substrate 31 advances through the interior of the groove 36 of the substrate 31 by dint of the capillarity and gets accumulated in the interior of the groove 36 of the substrate 31. Even when the quantity of the analyte in the periphery of the electrochemical sensor 30 inserted into the skin 6 decreases, if kept in the state where the analyte remains in the interior of the groove 36 of the substrate 31, it is feasible to restrain a risk against the depletion of the analyte on the surface of the working electrode 32A. Especially when the analyte is the interstitial fluid, since such a possibility exists that there is not the analyte quantity plentiful enough to flow in vivo, the depletion of the interstitial fluid on the surface of the working electrode 32A can be restrained by reserving the interstitial fluid in the interior of the groove 36 of the substrate 31.

A widthwise length and a depth of the groove 36 of the substrate 31 are determined so that the analyte is introduced into the interior of the groove 36 of the substrate 31 by dint of the capillarity and that the analyte advances through the interior of the groove 36 of the substrate 31 by dint of the capillarity. The widthwise length of the groove 36 of the substrate 31 is equal to or larger than, e.g., 0.05 mm but equal to or smaller than 3 mm, preferably equal to or larger than 0.1 mm but equal to or smaller than 3 mm, and more preferably equal to or larger than, e.g., 0.1 mm but equal to or smaller than 1 mm. Furthermore, the depth of the groove 36 of the substrate 31 is equal to or larger than, e.g., 50 µm but equal to or smaller than 200 µm and preferably equal to or larger than 75 µm but equal to or smaller than 150 µm.

It is preferable that an internal surface of the groove 36 of the substrate 31 undergoes a hydrophilization process. A variety of known methods can be adopted as a method of the hydrophilization process. The hydrophilization process may be executed by using, e.g., VUV (vacuum ultraviolet rays) process, a plasma exposure process, a surface active agent coating process, etc. Further, the hydrophilization process may also be carried out by using the same method as the method described in Japanese Unexamined Patent Publication No. 2010-532269. The internal surface of the groove 36 of the substrate 31 is subjected to the hydrophilization process, thereby accelerating a degree of how much the interior of the groove 36 of the substrate 31 get wetted with the analyte and accelerating the advancement of the analyte through the interior of the groove 36 of the substrate 31. It is preferable that the hydrophilization process over the internal surface of the groove 36 of the substrate 31 is performed so that a contact angle with the analyte is smaller than, e.g., 30 degrees. If the internal surface of the groove 36 of the substrate 31 is hydrophilized in such an extent as to allow the analyte to advance through the interior of the groove 36 of the substrate 31, however, the contact angle with the analyte may be equal to or larger than 30 degrees.

It is preferable that the VUV process is conducted by use of, e.g., the ultraviolet rays (an excimer laser etc) having a wavelength of 172 nm with an intensity of 2.3 mW/cm² (in a case where a distance between the light source and the substrate 31 is 4 mm) for irradiation time that is equal to or longer than several dozens of seconds but equal to shorter than 30 minutes. The VUV process may use, without being limited to the ultraviolet rays having the wavelength of 172 nm, the ultraviolet rays having other wavelengths.

The second working example demonstrates the instance of forming the groove 36 in the substrate 31 in a way that extends from the end portion 31B of the substrate 31 to the end portion 31A of the substrate 31. The present embodiment is not, however, limited to this formation, and the groove 36 may be formed in the substrate 31 so as to extend at a predetermined distance from the end portion 31B of the substrate 31 in the longitudinal direction of the substrate 31. For example, the groove 36 may also be formed in a portion of the substrate 31, which is inserted into the skin 6.

### <First Modified Example>

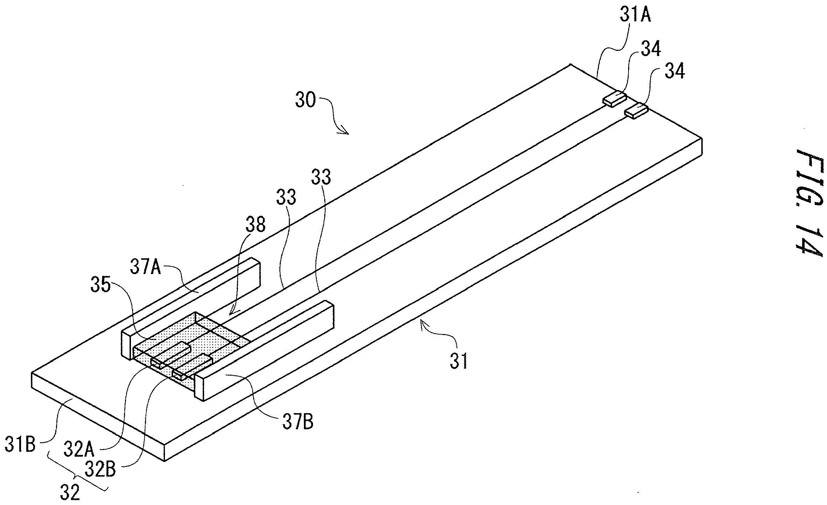

A first modified example of the second working example will be described. FIG. 14 is a perspective view of the whole electrochemical sensor 30 according to the first modified example of the second working example. A projection 37A and a projection 37B are formed on the substrate 31 in the longitudinal direction of the substrate 31. Further, a groove 38 is formed in the substrate 31 between the projection 37A and the projection 37B. The groove 38 of the substrate 31 is formed in a concave shape. The concave shape includes a quadrangular shape, a semicircular shape, a semielliptical shape, a pyramid shape, etc.

A resist pattern is formed on the substrate 31 by, e.g., the photolithography technology, and the substrate 31 is etched with the resist pattern serving as a mask, whereby the substrate 31 can be formed with the projection 37A, the projection 37B and the groove 38. Moreover, the substrate 31 can be formed with the projection 37A, the projection 37B and the groove 38 also by etching based on the laser beam machining.

The groove 38 may be formed in the substrate 31 in a manner that extends from the end portion 31B of the substrate 31 in the longitudinal direction of the substrate 31. That is, the substrate 31 is provided with the projection 37A and the projection 37B so that the projection 37A and the projection 37B abut on the end portion 31B of the substrate 31, and the groove 38 may be formed between the projection 37A and the projection 37B.

The groove 38 may be formed in the substrate 31 in a manner that extends from the end portion 31A of the substrate 31 in the longitudinal direction of the substrate 31. That is, the substrate 31 is provided with the projection 37A and the projection 37B so that the projection 37A and the projection 37B abut on the end portion 31A of the substrate 31, and the groove 38 may be formed between the projection 37A and the projection 37B.

The groove 38 may be formed in the substrate 31 in a way that extends from the end portion 31B of the substrate 31 up to the end portion 31A of the substrate 31 in the longitudinal direction of the substrate 31. Namely, the substrate 31 is provided with the projection 37A and the projection 37B so that the projection 37A and the projection 37B abut on the end portion 31B of the substrate 31 and that the projection 37A and the projection 37B abut on the end portion 31A of the substrate 31, and the groove 38 may be formed between the projection 37A and the projection 37B.

The electrode 32, the lead wires 33 and the external layer film 35 are provided in the interior of the groove 38 of the substrate 31. FIG. 14 illustrates an example in which a single working electrode 32A and a single counter electrode 32B are provided in the interior of the groove 38 of the substrate 31. The present embodiment is not, however, limited to this configuration, and a plurality of electrodes 32 may be provided in the interior of the groove 38 of the substrate 31. Further, a plurality of working electrodes 32A may be provided in the interior of the groove 38 of the substrate 31, and a plurality of counter electrodes 32B may also be provided in the interior of the groove 38 of the substrate 31.

In the case of inserting the electrochemical sensor 30 into the skin 6, the analyte within the skin 6 contacts the groove 38 of the substrate 31. The analyte coming into contact with the groove 38 of the substrate 31 is introduced into the interior of the groove 38 of the substrate 31 by dint of the capillarity. The analyte introduced into the interior of the groove 38 of the substrate 31 permeates the interior of the external layer film 35 and is thus supplied to the surface of the working electrode 32A.

A widthwise length and a depth of the groove 38 of the substrate 31 are determined so that the analyte is introduced into the interior of the groove 38 of the substrate 31 by dint of the capillarity and that the analyte advances through the interior of the groove 38 of the substrate 31 by dint of the capillarity. The widthwise length of the groove 38 of the substrate 31 is equal to or larger than, e.g., 0.05 mm but equal to or smaller than 3 mm, preferably equal to or larger than 0.1 mm but equal to or smaller than 3 mm, and more preferably equal to or larger than, e.g., 0.1 mm but equal to or smaller than 1 mm. Furthermore, the depth of the groove 38 of the substrate 31 is equal to or larger than, e.g., 50 µm but equal to or smaller than 200 µm and preferably equal to or larger than 75 µm but equal to or smaller than 150 µm.

It is preferable that an internal surface of the groove 38 of the substrate 31 undergoes a hydrophilization process. A variety of known methods can be adopted as a method of the hydrophilization process. The hydrophilization process may be executed by using, e.g., VUV (vacuum ultraviolet rays) process, a plasma exposure process, a surface active agent coating process, etc. Further, the hydrophilization process may also be carried out by using the same method as the method described in Japanese Unexamined Patent Publication No. 2010-532269. The internal surface of the groove 38 of the substrate 31 is subjected to the hydrophilization process, thereby accelerating a degree of how much the interior of the groove 38 of the substrate 31 get wetted with the analyte and accelerating the advancement of the analyte through the interior of the groove 38 of the substrate 31. It is preferable that the hydrophilization process over the internal surface of the groove 38 of the substrate 31 is performed so that a contact angle with the analyte is smaller than, e.g., 30 degrees. If the internal surface of the groove 38 of the substrate 31 is hydrophilized in such an extent as to allow the analyte to advance through the interior of the groove 38 of the substrate 31, however, the contact angle with the analyte may be equal to or larger than 30 degrees.

It is preferable that the VUV process is conducted by use of, e.g., the ultraviolet rays (an excimer laser etc) having a wavelength of 172 nm with an intensity of 2.3 mW/cm² (in a case where a distance between the light source and the substrate 31 is 4 mm) for irradiation time that is equal to or longer than several dozens of seconds but equal to shorter than 30 minutes. The VUV process may use, without being limited to the ultraviolet rays having the wavelength of 172 nm, the ultraviolet rays having other wavelengths.

### <Second Modified Example>

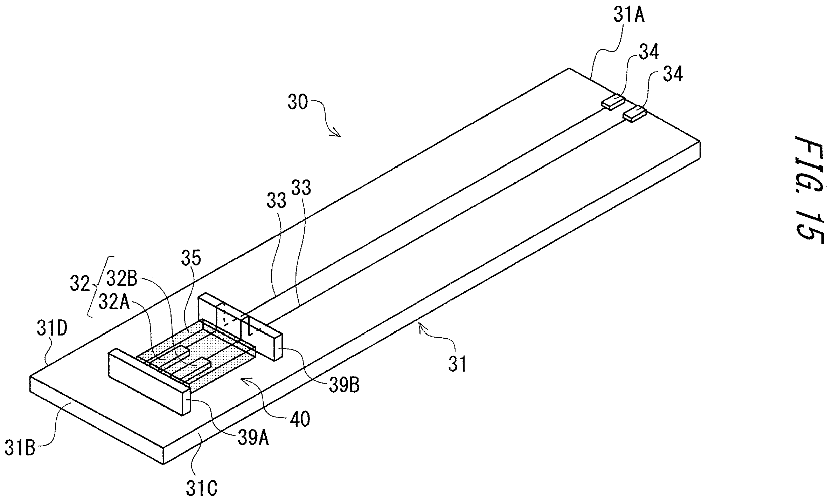

A second modified example of the second working example will be described. FIG. 15 is a perspective view of the whole electrochemical sensor 30 according to the second modified example of the second working example. A projection 39A and a projection 39B are formed on the substrate 31 in the widthwise direction of the substrate 31. Further, a groove 40 is formed in the substrate 31 between the projection 39A and the projection 39B. The groove 40 of the substrate 31 is formed in a concave shape. The concave shape includes a quadrangular shape, a semicircular shape, a semielliptical shape, a pyramid shape, etc.

A resist pattern is formed on the substrate 31 by, e.g., the photolithography technology, and the substrate 31 is etched with the resist pattern serving as a mask, whereby the substrate 31 can be formed with the projection 39A, the projection 39B and the groove 40. Moreover, the substrate 31 can be formed with the projection 39A, the projection 39B and the groove 40 also by etching based on the laser beam machining.

The groove 40 may be formed in the substrate 31 in a manner that extends from the end portion 31C of the substrate 31 in the widthwise direction of the substrate 31. That is, the substrate 31 is provided with the projection 39A and the projection 39B so that the projection 39A and the projection 39B abut on the end portion 31C of the substrate 31, and the groove 40 may be formed between the projection 39A and the projection 39B.

The groove 40 may be formed in the substrate 31 in a manner that extends from the end portion 31D of the substrate 31 in the widthwise direction of the substrate 31. That is, the substrate 31 is provided with the projection 39A and the projection 39B so that the projection 39A and the projection 39B abut on the end portion 31C of the substrate 31, and the groove 40 may be formed between the projection 39A and the projection 39B.

The groove 40 may be formed in the substrate 31 in a way that extends from the end portion 31C of the substrate 31 up to the end portion 31D of the substrate 31 in the widthwise direction of the substrate 31. Namely, the substrate 31 is provided with the projection 39A and the projection 39B so that the projection 39A and the projection 39B abut on the end portion 31C of the substrate 31 and that the projection 39A and the projection 39B abut on the end portion 31D of the substrate 31, and the groove 40 may be formed between the projection 39A and the projection 39B.

The electrode 32, the lead wires 33 and the external layer film 35 are provided in the interior of the groove 40 of the substrate 31. FIG. 15 illustrates an example in which a single working electrode 32A and a single counter electrode 32B are provided in the interior of the groove 40 of the substrate 31. The present embodiment is not, however, limited to this configuration, and a plurality of electrodes 32 may be provided in the interior of the groove 40 of the substrate 31. Further, a plurality of working electrodes 32A may be provided in the interior of the groove 40 of the substrate 31, and a plurality of counter electrodes 32B may also be provided in the interior of the groove 40 of the substrate 31.

In the case of inserting the electrochemical sensor 30 into the skin 6, the analyte within the skin 6 contacts the groove 40 of the substrate 31. The analyte coming into contact with the groove 40 of the substrate 31 is introduced into the interior of the groove 40 of the substrate 31 by dint of the capillarity. The analyte introduced into the interior of the groove 40 of the substrate 31 permeates the interior of the external layer film 35 and is thus supplied to the surface of the working electrode 32A.

A widthwise length and a depth of the groove 40 of the substrate 31 are determined so that the analyte is introduced into the interior of the groove 40 of the substrate 31 by dint of the capillarity and that the analyte advances through the interior of the groove 40 of the substrate 31 by dint of the capillarity. The widthwise length of the groove 40 of the substrate 31 is equal to or larger than, e.g., 0.05 mm but equal to or smaller than 3 mm, preferably equal to or larger than 0.1 mm but equal to or smaller than 3 mm, and more preferably equal to or larger than, e.g., 0.1 mm but equal to or smaller than 1 mm. Furthermore, the depth of the groove 40 of the substrate 31 is equal to or larger than, e.g., 50 µm but equal to or smaller than 200 µm and preferably equal to or larger than 75 µm but equal to or smaller than 150 µm.

It is preferable that an internal surface of the groove 40 of the substrate 31 undergoes a hydrophilization process. A variety of known methods can be adopted as a method of the hydrophilization process. The hydrophilization process may be executed by using, e.g., VUV (vacuum ultraviolet rays) process, a plasma exposure process, a surface active agent coating process, etc. Further, the hydrophilization process may also be carried out by using the same method as the method described in Japanese Unexamined Patent Publication No. 2010-532269. The internal surface of the groove 40 of the substrate 31 is subjected to the hydrophilization process, thereby accelerating a degree of how much the interior of the groove 40 of the substrate 31 get wetted with the analyte and accelerating the advancement of the analyte through the interior of the groove 40 of the substrate 31. It is preferable that the hydrophilization process over the internal surface of the groove 40 of the substrate 31 is performed so that a contact angle with the analyte is smaller than, e.g., 30 degrees. If the internal surface of the groove 40 of the substrate 31 is hydrophilized in such an extent as to allow the analyte to advance through the interior of the groove 40 of the substrate 31, however, the contact angle with the analyte may be equal to or larger than 30 degrees.

It is preferable that the VUV process is conducted by use of, e.g., the ultraviolet rays (an excimer laser etc) having a wavelength of 172 nm with an intensity of 2.3 mW/cm² (in a case where a distance between the light source and the substrate 31 is 4 mm) for irradiation time that is equal to or longer than several dozens of seconds but equal to shorter than 30 minutes. The VUV process may use, without being limited to the ultraviolet rays having the wavelength of 172 nm, the ultraviolet rays having other wavelengths.

### DESCRIPTION OF THE REFERENCE NUMERALS AND SYMBOLS

1 composition continuous measuring apparatus
2 housing
3 circuit board
4, 30 electrochemical sensor
5 bonding film
6 skin
10 cover
11 base plate
21, 31 substrate
12, 24, 34 terminal
21A, 21B, 21C, 21D, 31A, 31B, 31C, 31D end portion
22, 32 electrode
22A, 32A working electrode
22A, 32B counter electrode
23, 33 lead wire
25, 35 external layer film
26, 27A, 27B, 28, 29, 36, 38, 40 groove
37A, 37B, 39A, 39B projection

## Claims

1. An electrochemical sensor comprising:
a substrate (21, 31);
an electrode (22, 32) provided on the substrate in a direction; a reagent enzyme formed on a surface of the electrode;
an external layer film (25, 35) provided on the substrate so as to cover the electrode and the reagent enzyme; and
a groove (26, 36) formed in at least a part of the substrate in the direction in which the electrode is provided on the substrate,
**characterised in that** the external layer film (25, 35) is structured to allow an analyte to permeate inside the external layer film; and **in that**
the groove has an interior configured for the introduction of the analyte and for advancement of the analyte to contact the external layer film covering the electrode, so as to permeate the interior of the external layer film and be supplied onto the surface of the electrode via the external layer film.

2. The electrochemical sensor according to claim 1, wherein the electrode (22) is provided on the substrate (21) so that at least part of the electrode is positioned upwardly of the groove.

3. The electrochemical sensor according to claim 1, wherein the electrode (22) is provided, on the substrate (21), adjacent to the groove (26).

4. The electrochemical sensor according to any one of claims 1 through 3, wherein the substrate (21) is formed with a plurality of grooves (26).

5. The electrochemical sensor according to any one of claims 1 through 4, wherein a plurality of electrodes (22) is provided on the substrate (21).

6. The electrochemical sensor according to any one of claims 1 through 5, wherein the groove (26) according to claim 1 is formed in a concave shape.

7. The electrochemical sensor according to any one of claims 1 through 6, wherein the groove (26) according to claim 1 is formed in a way that extends from an end portion (21B) of the substrate (21) toward the direction of the electrode (22) of the substrate.

8. The electrochemical sensor according to claim 4, wherein at least two grooves (26) in the plurality of grooves are connected to each other.

9. The electrochemical sensor according to any one of claims 1 through 8, wherein the electrochemical sensor configured to be used in the way of being subcutaneously indwelled.

10. An electrochemical sensor according to claim 1; wherein the electrode (32), the external layer film (35) and the reagent enzyme are provided in an interior of the groove (36).

11. The electrochemical sensor according to claim 10, wherein the groove (36) is formed in a concave shape.

12. The electrochemical sensor according to any one of claims 10 or 11, wherein the groove (36) is formed in a way that extends from one end portion (31A) of the substrate (31) up to the other end portion (31B) of the substrate.

13. The electrochemical sensor according to any one of claims 10 through 12, wherein the groove (36) is formed in a longitudinal direction of the substrate (31).

14. The electrochemical sensor according to any one of claims 10 through 13, wherein the electrochemical sensor configured to be used in the way of being subcutaneously indwelled.

## Patentansprüche

1. Elektrochemischer Sensor, umfassend:
ein Substrat (21, 31);
eine Elektrode (22, 32), die auf dem Substrat in einer Richtung bereitgestellt ist;
ein Reagenzenzym, das auf einer Oberfläche der Elektrode ausgebildet ist;
einen Außenschichtüberzug (25, 35), der auf dem Substrat bereitgestellt ist, damit die Elektrode und das Reagenzenzym bedeckt sind; und
eine Nut (26, 36), die in mindestens einem Teil des Substrats in der Richtung, in der die Elektrode auf dem Substrat bereitgestellt ist, ausgebildet ist,
**dadurch gekennzeichnet, dass** der Außenschichtüberzug (25, 35) strukturiert ist, um einem Analyten zu ermöglichen, in die Außenschicht einzudringen; und
dadurch, dass die Nut einen Innenraum aufweist, der zur Einführung des Analyten und zum Vorschub des Analyten, um den Außenschichtüberzug, der die Elektrode bedeckt, zu berühren, damit er in den Innenraum des Außenschichtüberzugs eindringt und über den Außenschichtüberzug auf die Oberfläche der Elektrode aufgebracht wird, konfiguriert ist.

2. Elektrochemischer Sensor nach Anspruch 1, wobei die Elektrode (22) auf dem Substrat (21) bereitgestellt ist, sodass zumindest ein Teil der Elektrode oberhalb der Nut angeordnet ist.

3. Elektrochemischer Sensor nach Anspruch 1, wobei die Elektrode (22) benachbart zu der Nut (26) auf dem Substrat (21) bereitgestellt ist.

4. Elektrochemischer Sensor nach einem der Ansprüche 1 bis 3, wobei das Substrat (21) mit einer Vielzahl von Nuten (26) ausgebildet ist.

5. Elektrochemischer Sensor nach einem der Ansprüche 1 bis 4, wobei auf dem Substrat (21) eine Vielzahl von Elektroden (22) bereitgestellt ist.

6. Elektrochemischer Sensor nach einem der Ansprüche 1 bis 5, wobei die Nut (26) nach Anspruch 1 in einer konkaven Form ausgebildet ist.

7. Elektrochemischer Sensor nach einem der Ansprüche 1 bis 6, wobei die Nut (26) nach Anspruch 1 in einer Weise ausgebildet ist, dass sie sich von einem Endabschnitt (21B) des Substrats (21) in die Richtung der Elektrode (22) auf dem Substrat erstreckt.

8. Elektrochemischer Sensor nach Anspruch 4, wobei mindestens zwei Nuten (26) in der Vielzahl von Nuten miteinander verbunden sind.

9. Elektrochemischer Sensor nach einem der Ansprüche 1 bis 8, wobei der elektrochemische Sensor konfiguriert ist, als subkutan verweilend verwendet zu werden.

10. Elektrochemischer Sensor nach Anspruch 1, wobei die Elektrode (32), der Außenschichtüberzug (35) und das Reagenzenzym in einem Innenraum der Nut (36) bereitgestellt sind.

11. Elektrochemischer Sensor nach Anspruch 10, wobei die Nut (36) in einer konkaven Form ausgebildet ist.

12. Elektrochemischer Sensor nach einem der Ansprüche 10 bis 11, wobei die Nut (36) in einer Weise ausgebildet ist, dass sie sich von einem Endabschnitt (31A) des Substrats (31) bis zu dem anderen Endabschnitt (31B) des Substrats erstreckt.

13. Elektrochemischer Sensor nach einem der Ansprüche 10 bis 12, wobei die Nut (36) in einer Längsrichtung des Substrats (31) ausgebildet ist.

14. Elektrochemischer Sensor nach einem der Ansprüche 10 bis 13, wobei der elektrochemische Sensor konfiguriert ist, als subkutan verweilend verwendet zu werden.

## Revendications

1. Capteur électrochimique comprenant :
un substrat (21, 31) ;
une électrode (22, 32) disposée sur le substrat dans une direction ;
un enzyme réactif formé sur une surface de l'électrode ;
un film à couche externe (25, 35) appliqué sur le substrat de manière à couvrir l'électrode et l'enzyme réactif ; et
une rainure (26, 36) formée dans au moins une partie du substrat dans la direction dans laquelle l'électrode est disposée sur le substrat,
**caractérisé en ce que** le film à couche externe (25, 35) est structuré pour permettre à un analyte de pénétrer dans le film à couche externe ; et **en ce que** la rainure a un intérieur configuré pour l'introduction de l'analyte et pour l'avancement de l'analyte afin qu'il vienne en contact avec le film à couche externe recouvrant l'électrode de manière à pénétrer à l'intérieur du film à couche externe et d'être acheminé sur la surface de l'électrode via le film à couche externe.

2. Capteur électrochimique selon la revendication 1, dans lequel l'électrode (22) est disposée sur le substrat (21) de sorte qu'au moins une partie de l'électrode soit positionnée vers le haut de la rainure.

3. Capteur électrochimique selon la revendication 1, dans lequel l'électrode (22) est disposée de manière adjacente à la rainure (26) sur le substrat (21).

4. Capteur électrochimique selon l'une quelconque des revendications 1 à 3, dans lequel le substrat (21) présente une pluralité de rainures (26).

5. Capteur électrochimique selon l'une quelconque des revendications 1 à 4, dans lequel une pluralité d'électrodes (22) est appliquée sur le substrat (21).

6. Capteur électrochimique selon l'une quelconque des revendications 1 à 5, dans lequel la rainure (26) selon la revendication 1 présente une forme concave.

7. Capteur électrochimique selon l'une quelconque des revendications 1 à 6, dans lequel la rainure (26) selon la revendication 1 est formée de manière à s'étendre d'une partie d'extrémité (21b) du substrat (21) dans la direction de l'électrode (22) du substrat.

8. Capteur électrochimique selon la revendication 4, dans lequel au moins deux rainures (26) de la pluralité de rainures sont raccordées l'une à l'autre.

9. Capteur électrochimique selon l'une quelconque des revendications 1 à 8, dans lequel le capteur électrochimique est configuré pour être utilisé de manière à être incorporé par voie sous-cutanée.

10. Capteur électrochimique selon la revendication 1, dans lequel l'électrode (32), le film à couche externe (35) et l'enzyme réactif sont disposés à l'intérieur de la rainure (36).

11. Capteur électrochimique selon la revendication 10, dans lequel la rainure (36) présente une forme concave.

12. Capteur électrochimique selon l'une quelconque des revendications 10 ou 11, dans lequel la rainure (36) est formée de manière à s'étendre d'une partie d'extrémité (32A) du substrat (31) jusqu'à l'autre partie d'extrémité (31B) du substrat.

13. Capteur électrochimique selon l'une quelconque des revendications 10 à 12, dans lequel la rainure (36) est formée dans la direction longitudinale du substrat (31).

14. Capteur électrochimique selon l'une quelconque des revendications 10 à 13, dans lequel le capteur électrochimique est configuré pour être utilisé de manière à être incorporé par voie sous-cutanée.
